# EUROPÄISCHE PATENTANMELDUNG

(11) **EP 0 558 961 A2**
(43) Veröffentlichungstag der Anmeldung: **08.09.1993**
(21) Anmeldenummer: 93102048.1
(22) Anmeldetag: 10.02.1993
(51) Int. Cl.: C07K 9/00, A61K 37/02

(54) **Glycopeptid-Derivate, Verfahren zu ihrer Herstellung und diese Verbindungen enthaltende pharmazeutische Mittel**

(30) Priorität: 05.03.1992 DE 4206858
(71) Anmelder: BEHRINGWERKE Aktiengesellschaft, 35001 Marburg (DE)
(72) Erfinder: Kolar, Cenek, W-3550 Marburg (DE); Stüber, Werner, W-3551 Lahntal 3 (DE)

(57) **Zusammenfassung**

Es werden O- und N-Glycopeptid-Derivate der Formel I 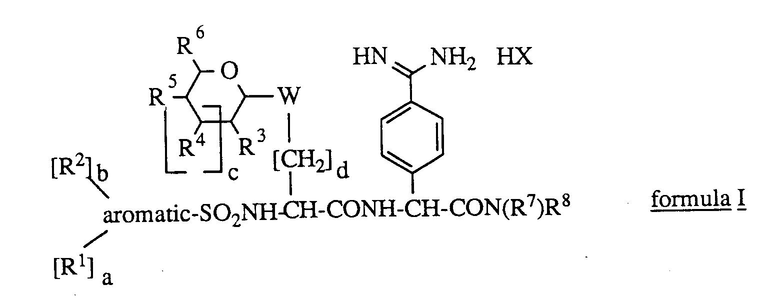
worin
- Aromat: Benzol-, Naphthalin-, Chroman-, Chromen- oder Cumaron-Rest,
- a: 1 bis 5, b 0 bis 4, c 0 oder 1, d 1 oder 2 und die Reste
- R¹: H oder (C₁-C₃)-Alkyl,
- R²: H, (C₁-C₃)-Alkyl oder (C₁-C₃)-Alkyloxy,
- R³: H, OH, (C₁-C₃)-Alkyloxy, NH₂, NH-(C₁-C₆)-Alkanoyl, NH-Benzoyl, NH-SO₃H oder NH-Acylrest einer natürlichen N-acetylierten Aminosäure,
- R⁴: H, OH, oder (C₁-C₃)-Alkyloxy,
- R⁵: H, OH, (C₁-C₃)-Alkyloxy, Fluor, Chlor oder Brom,
- R⁶: H, CH₃, CH₂OH, CH₂O-(C₁-C₆)-Alkanoyl, CH₂NHCOCH₃ oder CH₂NH-SO₃H
- R⁵ und R⁶: zusammen O-CH₂-O-CH₂, O-CH(CH₃)-O-CH₂ oder O-C(CH₃)₂-O-CH₂,
- R⁷: Hydroxy-(C₂-C₄)-alkyl oder (C₂-C₄)-Alkyloxy-(C₂-C₄)-alkyl,
- R⁸: H oder (C₁-C₆)-Alkyl oder
- R⁷-N-R⁸: zusammen einen Pyrrolidin-, Piperidin- oder Morpholinring bilden, der mit den Substituenten HO, HOCH₂, CH₃ oder COOH werden kann,
- W: -O-, -CONH- oder -C₆H₄CONH- und
- H-X: HCl, (C₁-C₇)-Alkansäure oder eine physiologisch verträgliche anorganische oder organische Säure, sind,
beschrieben, Verfahren zu ihrer Herstellung und ein pharmazeutisches Mittel, in dem diese neuen Verbindungen enthalten sind. Diese Arzneimittel können besonders zur Therapie thrombotischer Erkrankungen verwendet werden.

## Beschreibung

Die Erfindung betrifft O- und N-Glycopeptid-Derivate, Verfahren zu ihrer Herstellung und ein pharmazeutisches Mittel, in dem diese neuen Verbindungen enthalten sind.

Der Hauptvorgang bei der Gerinnung von Blut besteht darin, daß das lösliche Protein Fibrinogen in das unlösliche Protein Fibrin überführt wird. Hierfür ist ein proteolytischer Katalysator, das Thrombin, notwendig. Durch Proteolyse wird das Proenzym Prothrombin in Thrombin umgewandelt. Diese Proteolyse wird durch extra- oder intravasale Aktivierungsprozesse in Gang gesetzt. Das hämostatische Gleichgewicht wird nicht nur durch Aktivatoren, sondern auch durch Inhibitoren sowohl der Gerinnung als auch der Fibrinolyse reguliert. Der wichtigste physiologische Inhibitor der Gerinnung ist Antithrombin III (AT III). Es inaktiviert das Thrombin zunächst nur geringfügig und erst nach einer gewissen Anlaufzeit in größerem Umfange. AT III ist somit direkt an einem Abfangmechanismus gegen eine überschießende Thrombin-Aktivierung beteiligt. Eine Reihe von pathophysiologischen Bedingungen führt zu einem Verbrauch von AT III und somit zu einem erhöhtem Thromboserisiko. Das Krankheitsbild der disseminierten intravasalen Gerinnung, zuweilen noch als Verbrauchs-Koagulopathie bezeichnet, tritt häufig nach Operationen und bei Schockzuständen auf. In vielen Fällen treten dabei lebensbedrohliche Blutgerinnsel auf. Zur Therapie thrombotischer Erkrankungen werden Antikoagulantien, wie AT III und Heparin, und neuerdings auch Hirudin und zur Prophylaxe Cumarin-Derivate eingesetzt. Die Verwendung der hier aufgeführten Antikoagulantien ist mit Nachteilen verbunden. Heparin, das kein einheitliches Molekül ist, wirkt nur in der Gegenwart des Cofaktors AT III. Es kann nur parenteral appliziert werden. Hirudin bzw. AT III müssen aus biologischem Material isoliert oder gentechnologisch hergestellt werden. Die Langzeitprophylaxe mit den Vitamin K-antagonistisch wirkenden Cumarinen ist mit Nebenwirkungen, wie hämorrhagische Hautnekrosen, Haarausfall und Nausea verbunden. Zur Therapie thrombotischer Erkrankungen wurden auch niedermolekulare Antikoagulantien entwickelt, die direkt auf das proteolytisch aktive Zentrum des Thrombins einwirken. In der EPA 293881 A2 und WO 89/03223 sind Serin-Protease-Inhibitoren, die sich von der Peptid-Boronsäure ableiten, beschrieben. Diese Verbindungen inhibieren nicht nur Thrombin sondern auch andere Serin-Proteasen, wie z. B. Trypsin. G. Wagner et al. [DD 155954 - CA 98(13): 107770b] und B. Kaiser et al. (Biomed. Biochim. Acta 44, 7/8 (1985)1201) beschreiben die pharmakologischen Eigenschaften eines wirksamen (Thrombin-Bindungskonstante: Ki = 6 nmol/L) Thrombin-Inhibitors N-α-(2-Naphthalinsulfonylglycyl)-4-amidinophenyl-alanin piperidid (NAPAP). In der EP 0236 163 und EP 0236 164 sind weitere Derivate dieser Struktur beschrieben, in denen vor allem der aromatische Sulfonamid-Teil modifiziert und die Aminosäure Glycin durch andere Aminosäuren ausgetauscht sind. Der Nachteil der NAPAP-Verbindung sowie der beanspruchten Derivate liegt in der pharmakologischen Verträglichkeit - hierbei wird vor allem der Blutdruckabfall und die Freisetzung von Histamin beobachtet, der zu geringen Thrombinspezifität sowie der geringen Löslichkeit.
Überraschenderweise zeigte sich, daß die N-Glycopeptid-Derivate, wie 2-N-(4-Methoxy 2,3,6-trimethylbenzolsulfonyl-(4-N-(β-D-glucuronyl- bzw. β-D-glucopyranosyl)-L-asparaginyl-4-amidino-D-phenylalanin piperidid sehr wirksame (Bindungskonstante: Ki = 0.08 nmol/L) und spezifische Thrombininhibitoren darstellen und daß ihre Wasserlöslichkeit beträchtlich verbessert wurde.
Ausgehend von dieser Erkenntnis hatte es sich die vorliegende Erfindung zur Aufgabe gestellt, neue O- und N-Glycopeptide und deren Derivate herzustellen, und deren Nützlichkeit als Antikoagulantien zu überprüfen.
Gelöst wurde diese Aufgabe durch die Herstellung sowie die pharmakologische Wirkungsbestimmung der Verbindungen der allgemeinen Formel I.

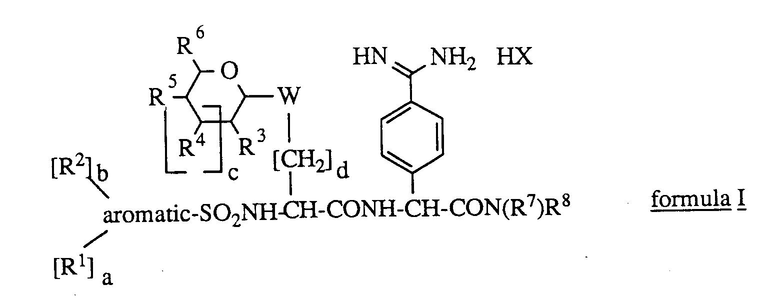

Gegenstand der Erfindung sind O- und N-Glycopeptid-Derivate der Formel I
worin
- Aromat: Benzol-, Naphthalin-, Chroman-, Chromen- oder Cumaron-Rest,
- a: 1 bis 5, b 0 bis 4, c 0 oder 1, d 1 oder 2 und die Reste
- R¹: H oder (C₁-C₃)-Alkyl,
- R²: H, (C₁-C₃)-Alkyl oder (C₁-C₃)-Alkyloxy,
- R³: H, OH, (C₁-C₃)-Alkyloxy, NH₂, NH-(C₁-C₆)-Alkanoyl, NH-Benzoyl, NH-SO₃H oder NH-Acylrest einer natürlichen N-acetylierten Aminosäure,
- R⁴: H, OH, oder (C₁-C₃)Alkyloxy,
- R⁵: H, OH, (C₁-C₃)-Alkyloxy, Fluor, Chlor oder Brom,
- R⁶: H, CH₃, CH₂OH, CH₂O-(C₁-C₆)-Alkanoyl, CH₂NHCOCH₃ oder CH₂NH-SO₃H
- R⁵ und R⁶: zusammen O-CH₂-O-CH₂, O-CH(CH₃)-O-CH₂ oder O-C(CH₃)₂-O-CH₂,
- R⁷: Hydroxy-(C₂-C₄)-alkyl oder (C₂-C₄)-Alkyloxy-(C₂-C₄)-alkyl,
- R⁸: H oder (C₁-C₆)-Alkyl oder
- R⁷-N-R⁸: zusammen einen Pyrrolidin-, Piperidin- oder Morpholinring bilden, der mit den Substituenten HO, HOCH₂, CH₃ oder COOH werden kann,
- W: -O-, -CONH- oder -C₆H₄CONH- und
- H-X: HCl, (C₁-C₇)-Alkansäure oder eine physiologisch verträgliche anorganische oder organische Säure, sind.

Bevorzugt werden im Rahmen der Erfindung Verbindungen der allgemeinen Formel I,
worin
- Aromat: Benzol-, Naphthalin- oder Chroman-Rest,
- a: 1 bis 4, b 0 bis 2, c 0 oder 1, d 1 oder 2 und die Reste
- R¹: H oder CH₃,
- R²: H, CH₃ oder CH₃O,
- R³: H, OH, (C₁-C₃)-Alkyloxy, NH₂, NH-Acetyl, NH-Benzoyl, NH-SO₃H oder NH-Acylrest von N-Acetyl-glycin oder N-Acetyl-alanin,
- R⁴: H oder OH,
- R⁵: H oder OH,
- R⁶: H, CH₃, CH₂OH, CH₂O-(C₁-C₆)-Alkanoyl, CH₂NHCOCH₃ oder CH₂NH-SO₃H
- R⁵ und R⁶: zusammen O-CH₂-O-CH₂ oder O-CH(CH₃)-O-CH₂,
- R⁷: Hydroxypropyl oder Ethoxypropyl,
- R⁸: H oder (C₁-C₆)-Alkyl oder
- R⁷-N-R⁸: zusammen einen Pyrrolidin-, Piperidin- oder Morpholinring bilden,
- W: -O-, -CONH- oder -C₆H₄CONH- und
- H-X: HCl, (C₁-C₆)-Alkansäure oder eine pharmakologisch vertretbare anorganische oder organische Säure,
sind.

Die erfindungsgemaßen Verbindungen der Formel I sind dadurch herstellbar, daß man eine Verbindung der Formel II, 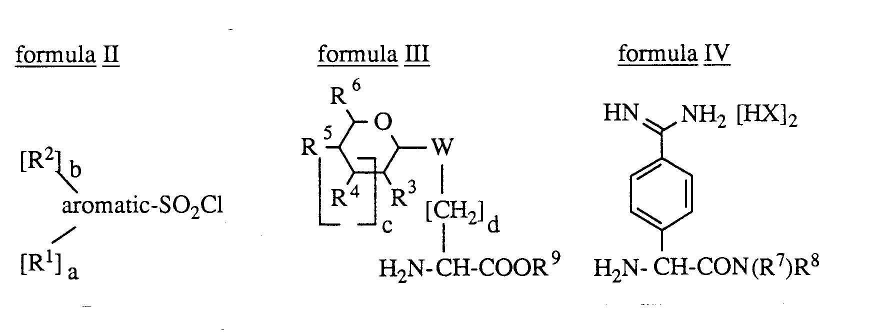
worin
- Aromat: Benzol-, Naphthalin-, Chroman-, Chromen- oder Cumaron-Rest,
- a: 1 bis 5, b 0 bis 4, und die Reste
- R¹: H oder (C₁-C₃)-Alkyl,
- R²: H, (C₁-C₃)-Alkyl oder (C₁-C₃)-Alkyloxy
bedeuten,
mit einem O- oder N-Glycosid der Formel III,
worin
- c: 0 oder 1, d 1 oder 2
- R³: H, O-Schutzgruppe, NH-Schutzgruppe, NH-SO₃H, (C₁-C₃)-Alkyloxy, NH-(C₁-C₆)-Alkanoyl, NH-Benzoyl, NH-SO₃H oder NH-Acylrest einer natürlichen N-acetylierten Aminosäure,
- R⁴: H, O-Schutzgruppe oder (C₁-C₃)-Alkyloxy,
- R⁵: H, O-Schutzgruppe, (C₁-C₃)-Alkyloxy, Fluor, Chlor oder Brom,
- R⁶: H, CH₃, CH₂-O-Schutzgruppe, CH₂O-(C₁-C₆)-Alkanoyl, CH₂NHCOCH₃ oder CH₂NH-SO₃H,
- R⁵ und R⁶: zusammen O-CH₂-O-CH₂, O-CH(CH₃)-O-CH₂ oder O-C(CH₃)₂-O-CH₂,
- R⁹: Methyl-, tert.-Butyl-, Allyl- oder Benzyl-Schutzgruppe und
- W: -O-, -CONH- oder -C₆H₄CONH-,
sind.
in Gegenwart einer organischen Base und eines organischen Losungsmittels zu einer Sulfonamid-Verbindung der Formel V, 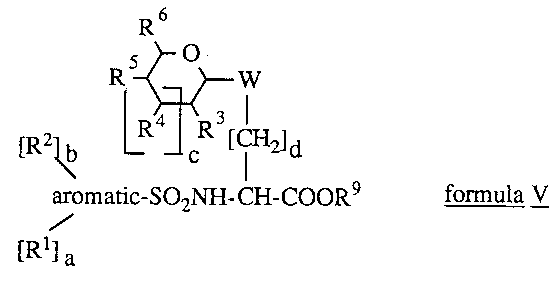
wobei
a, b, c und d und die Reste ihre vorher genannte Bedeutung beibehalten, umsetzt, in dem Produkt selektiv den Rest R⁹ hydrolytisch mit HCl/Essigsäure oder Trifluoressigsäure/Wasser oder hydrogenolytisch in Gegenwart von Palladium/Kohle und (C₁-C₄)-Alkohols, Essigsäure oder Ethylacetat als Lösungsmittels abspaltet, und das entstandene Produkt, worin R⁹ ein Wasserstoffatom ist, mit einem Phenylalaninderivat der Formel IV,
worin
- R⁷: Hydroxy-(C₂-C₄)-alkyl oder (C₂-C₄)-Alkyloxy-(C₂-C₄)-alkyl,
- R⁸: H oder (C₁-C₆)-Alkyl oder
- R⁷-N-R⁸: zusammen einen Pyrrolidin-, Piperidin- oder Morpholinring bilden,
- W: -O-, -CONH- oder -C₆H₄CONH- und
- H-X: HCl, HI, CF₃COOH sind,
nach einem in der Peptidchemie üblichen Kondensationsverfahren zu einer Verbindung der Formel I,
wobei
a, b, c und d und die Reste ihre vorher genannte Bedeutung beibehalten, umsetzt und in dem Produkt nach in der Kohlenhydrat- oder Peptidchemie üblichen Verfahren die Schutzgruppen entfernt, wobei ein weiteres Produkt der Formel I,
worin
- Aromat: Benzol-, Naphthalin-, Chroman-, Chromen- oder Cumaron-Rest,
- a: 1 bis 5, b 0 bis 4, c 0 oder 1, d 1 oder 2 und die Reste
- R¹: H oder (C₁-C₃)-Alkyl,
- R²: H, (C₁-C₃)-Alkyl oder (C₁-C₃)-Alkyloxy,
- R³: H, OH, (C₁-C₃)-Alkyloxy, NH₂, NH-(C₁-C₆)-Alkanoyl, NH-Benzoyl, NH-SO₃H oder NH-Acylrest einer natürlichen N-acetylierten Aminosäure,
- R⁴: H, OH, oder (C₁-C₃)-Alkyloxy,
- R⁵: H, OH, (C₁-C₃)-Alkyloxy, Fluor, Chlor oder Brom,
- R⁶: H, CH₃, CH₂OH, CH₂O-(C₁-C₆)-Alkanoyl, CH₂NHCOCH₃ oder CH₂NH-SO₃H,
- R⁵ und R⁶: zusammen O-CH₂-O-CH₂, O-CH(CH₃)-O-CH₂ oder O-C(CH₃)₂-O-CH₂,
- R⁷: Hydroxy-(C₂-C₄)-alkyl oder (C₂-C₄)-Alkyloxy-(C₂-C₄)-alkyl,
- R⁸: H oder (C₁-C₆)-Alkyl oder
- R⁷-N-R⁸: zusammen einen Pyrrolidin-, Piperidin- oder Morpholinring bilden und
- H-X: HCl, (C₁-C₆)-Alkansäure oder eine pharmakologisch vertretbare anorganische oder organische Säure, sind,
entsteht, und das so erhaltene Glycopeptid-Derivat gegebenenfalls in sein physiologisch verträgliches Salz überführt.
Die Kondensation nach dem erfindungsgemäßen Verfahren erfolgt nach den allgemeinen Methoden der Peptidchemie, bevorzugt nach der Methode der gemischten Anhydride, über Aktivester, Azide oder nach der Carbodiimidmethode, insbesondere unter Zusatz reaktionsbeschleunigender und racemisierungsverhindernder Substanzen wie z. B. 1-Hydroxy-benzotriazol (HOBt), N-Hydroxysuccinimid, N-Hydroxy-5-norbornen-2,3-dicarboximid, ferner unter Verwendung aktivierter Derivate des 1-Hydroxybenzotriazols oder Anhydriden von Phosphor-, Phosphon- und Phosphinsäuren bei einer Reaktionstemperatur zwischen -5^{o}C und 40°C. Geeignete Lösungsmittel dafür sind Dimethyl formamid (DMF), Dimethylacetamid (DMA), Hexamethylphosphorsäuretriamid (HMPT), N-Methylpyrrolidon oder Dimethylsulfoxid (DMSO). Sofern die Löslichkeit der Komponenten es erlaubt, können auch Lösungsmittel wie Methylenchlorid, Chloroform oder Dichlorethylen eingesetzt werden. Die genannten Methoden sind z. B. in Meienhofer-Gross: "The Peptides", Academic Press, Vol. I, (1979) beschrieben.
Die Auswahl der in der Kohlenhydrat- oder Peptidchemie üblichen Schutzgruppen für Amino-, Hydroxy- oder Carboxygruppen wird von der Synthesestrategie sowie der Art der Kupplungsbedingungen bestimmt. Unter den in der Kohlenhydratchemie üblichen Schutzgruppen für die Hydroxygruppen versteht man z.B. die (C₁-C₁₀)-Acylschutzgruppen wie (C₁-C₆)-Alkanoyl (z. B. Acetylgruppe oder Mono-, Di- oder Trihalogenacetyl mit Halogen Fluor oder Chlor), Benzoyl-, Methoxybenzoyl- oder p-Nitrobenzoyl-, sowie gegebenenfalls modifizierte Methyl-, Methoxymethyl-, Allyl-, Benzyl-, Tetrahydropyranyl-, Benzyliden-, Isopropyliden- oder Trityl-Gruppe, wobei hier die Acylschutzgruppen, insbesondere die Acetylgruppe, bevorzugt sind.
Zum Schutz der Aminogruppen werden Adamantyloxycarbonyl- (Adoc), Benzyloxy carbonyl- (Z), p-Nitrobenzyloxycarbonyl-(pNBz-), 9-FIuorenylmethoxycarbonyl-(Fmoc-), tert.-Butyloxycarbonyl- (BOC-) oder α,α-Dimethyl-3,5-dimethoxybenzyloxycarbonyl- (DDZ-) oder Trifluoracetyl- (TFAc-) -Gruppen eingesetzt. Zum Schutz der Carboxygruppen werden (C₁-C₄)-Alkyl-, Allyl- (All-) oder Benzyl-(Bn-)-Gruppen verwendet.
Die Schutzgruppen können nach üblichen Verfahren abgespalten werden, je nach Art der jeweiligen Gruppe zum Beispiel mit Trifluoroessigsäure, HCl oder HBr in Essigsäure oder durch milde Reduktion, zum Beispiel mit Wasserstoff und einem Katalysator, wie Palladium/Kohle oder Palladium/Bariumsulfat in (C₁-C₄)-Alkohol, Essigsäure oder Ethylacetat. Mit sekundären Aminen gegebenenfalls durch katalytische Hydrierung [z. B. R. Geiger und W. König in E. Gross und Meienhofer (Eds): The Peptides, Vol. 3, p. 24, Academic Press, 1981] läßt sich die Fmoc-Schutzgruppe abspalten.

Die O-Acyl- und COO-Alkyl-Schutzgruppen werden vorteilhaft im basischem Milieu zum Beispiel mit Natriummethylat, Natriumhydroxyd, Natriumcarbonat, Bariumoxid oder Kaliumcyanid oder mit organischen Basen, wie z. B. Hydrazin in (C₁-C₄)-Alkanol oder deren Mischungen mit Chloroform oder Wasser abgespalten.
Bei der Synthese der N-Glycopeptid-Bausteine der Formel III sind zwei polyfunktionelle Reaktionspartner (Kohlenhydrat und Aminosäure) zu verknüpfen. Beide müssen sich selektiv blockieren und deblockieren lassen. Je nach dem Typ der angestrebten glycosidischen Bindung (1,2-cis- oder 1,2-trans-O oder -N-Glycoside) ist es notwendig, geeignete Schutzgruppen für die Blockierung der Hydroxy-, Carboxy- oder Aminogruppen in die Glycosyl- sowie Aminosäure-Komponente einzuführen und Reaktionsbedingungen für den Verknüpfungsschritt auszuarbeiten, der stereoselektiv zu nur einem der beiden möglichen Anomere führt. Für die Herstellung der Glycopeptid-Bausteine der Formel III werden sowohl die in der Literatur bekannten Verfahren wie z. B. von A. Y. Khorlin et al. Carbohydr. Res., 85,201-208 (1980), R. Walczyna und J. Sokolowski, Carbohydr. Res., 180, 147-151(1988), A. Klemmer und M. Kohla, J. Carbohydr. Chem., 7(4), 785-797(1988), T. Takeda et al., Carbohydr. Res., 207, 71-79 (1990), W. Guenther und H. Kunz, Angew. Chem., 102/9, 1068 (1990), L. Urge et al., Tetrahedron Letters 32(29), 3445-3448 (1991) und L. Biondi et al., Int. J. Peptide Protein Res., 37, 112-121 (1991) beschrieben oder modifizierten Glycopeptidverfahren verwendet.
Im Hinblick auf die Therapie thrombotischer Erkrankungen wurde die pharmazeutische Verwendbarkeit der neuen Verbindungen der Formel I als Antikoagulantien in verschiedenen in vitro und in vivo Testsystemen untersucht. Zur Prüfung der Thrombin-inhibierenden Eigenschaften der neuen Verbindungen wurden Inhibitionskonstanten (Ki) in standardisierten Test mit chromogenen Substraten ermittelt. Die Spezifität der neuen Verbindungen gegenüber Serinproteasen wurde vor allem mit Thrombin und Trypsin untersucht. Die in vivo Wirksamkeit der Verbindungen der Formel I wurde durch die Ermittlung der akuten Toxizität (LD₅, LD₅₀) und partiellen Thromboplastinzeit (PTT) festgestellt.
Die chemische Stabilität der neuen Verbindungen wurde im Hinblick auf die perorale Anwendung (Magen- bzw. Darmpassage), mit starken Säuren und Laugen ermittelt. Die Stabilität der neuen Verbindungen gegenüber Degradationsenzymen wurde mit Trypsin, Chymotrypsin sowie indirekt mit Plasma, Leber- oder Darmhomogenaten durchgeführt. Die Erfindung betrifft weiterhin pharmazeutische Zubereitungen, welche ein Glycopeptid-Derivat der Formel I und ein pharmazeutisches Verdünnungsmittel, einen Lösungsvermittler oder einen Träger enthalten.

Von Vorteil ist die pharmazeutische Zubereitung, bei der eine Verbindung der Formel I in Form einer Emulsion oder einer Iiposomalen oder mizellären Präparation vorliegt. Als Hilfstoffe zur Herstellung einer pharmazeutisch unbedenklichen Zubereitung können Phospholipide, Cholesterin, Triglyceride, Detergentien wie z. B. Tween 80 oder deren Mischungen verwendet werden. Als Trägerstoffe eignen sich beispielsweise Albumine, Gelatine-Polymerisate wie Polygelin oder Stärke, Glucose, Lactose, Mannit oder Sorbit, die in einer physiologischen Lösung oder Festform vorliegen. Diese Zubereitungen enthalten eine therapeutisch wirksame Menge der Glycopeptid-Verbindung der Formel I. In den folgenden Beispielen wird die vorliegende Erfindung näher beschrieben, ohne sie hierbei einzuschränken.

### BEISPIELE

Die Struktur der folgenden Verbindungen wurde mittels 1H-NMR-, 13C-NMR- und IR-Spektroskopie sowie MS- und Elementaranalyse ermittelt.

### Beispiel 1

### Herstellung von Glycosylaminen:

### Methyl-(2,3,4-tri-O-acetyl-β-D-glucopyranosylamin)uronat (Verbindung 1)

Methyl-(2,3,4-tri-O-acetyl-β-D-glucopyranosylazid)-uronat (2.00 g)wurde in Ethylacetat/Methanol (2:1; 200 mL) gelöst. Nach der Zugabe von Palladium/Kohle (2.00 g) wurde die Mischung mit Triethylamin auf den pH-Wert von 7.5 eingestellt und dann eine Stunde hydriert. Die Reaktionsmischung wurde abfiltriert und die Lösung wurde in vacuo eingeengt. Ausbeute: 1.90 g. DC-Analyse: Rf = 0.35 (Dichlormethan/Aceton 2:1).

### 2,3,4,6-Tetra-O-acetyl-β-D-glucopyranosylamin (Verbindung 2)

Ausgehend von 2,3,4,6-Tetra-O-acetyl-β-D-glucopyranosylazid wurde die Titelverbindung wie oben beschrieben hergestellt.

### 2-Acetamido-2-desoxy-3,4,6-tri-O-acetyl-b-D-glucopyranosylamin (Verbindung 3)

Ausgehend von 2-Acetamido-2-desoxy-3,4,6-tri-O-acetyl-β-D-hexopyranosylazid wurde die Titelverbindung wie oben beschrieben hergestellt.

### Beispiel 2

### Herstellung von N-Glycosiden:

### 2-N-(Fmoc)-4-N-[methyl-(2,3,4-tri-O-acetyl-β-D-glucopyranosyl)uronat]-L-asparagin tert.-butylester (Verbindung 4)

2-N-(Fmoc)-L-asparaginsäure tert.-butylester (1.6 g), HOBt (0.90 g) und Dicyclohexylcarbodiimid (1.40 g) wurden in THF (100 mL) gelöst. Nach 1 h Rühren wurde die Mischung bei O^{o}C mit Verbindung 1 (1.4 g) versetzt. Die Mischung wurde 16 h bei Raumtemperatur gerührt und anschließend in vacuo eingeengt. Der Rückstand wurde mit Chloroform verdünnt, mit Wasser ausgeschüttelt, mit Natriumsulfat getrocknet und in vacuo eingedampft. Der Rückstand wurde säulenchromatographisch (Chloroform/Aceton 6:1) getrennt. Ausbeute: 2.00 g; DC-Analyse: Rf= 0.72 (Chloroform/Aceton 6:1)

### 2-N-(Z)-4-N-[Methyl-(2,3,4-tri-O-acetyl-β-D-glucopyranosyl)uronat]-L-asparagin tert.-butylester (Verbindung 5)

Ausgehend von 2-N-(Z)-L-asparaginsäure tert.-butylester (1.5 g) und der Verbindung 1(1.2 g) wurde die Titelverbindung wie oben beschrieben hergestellt. Ausbeute: 1.6 g.

### 2-N-(Z)-4-N-(2,3,4,6-tetra-O-acetyl-β-D-glucopyranosyl)-L-asparagin-tert.-butyl ester (Verbindung 6)

2-N-(Z)-L-asparaginsäure tert.-butylester (3.5 g), HOBt (1.6 g), und DCCI (2.5 g) wurden in DMF (160 mL) bei 0^{o}C gelöst und dann 1 h gerührt. Nach der Zugabe einer Lösung von Verbindung 2 (3.5 g) in DMF (50 mL) wurde die Reaktionsmischung 35 h bei Raumtemteratur gerührt, dann abfiltriert und eingedampft. Der Rückstand wurde in Dichlormethan gelöst, mit Wasser ausgewaschen und die organische Phase wurde über Natriumsulfat getrocknet und in vacuo eingeengt. Das Rohprodukt wurde säulenchromatographisch über Kieselgel (220 g) mit Dichlormethan und Aceton 5:1 gereinigt. Ausbeute: 3.6 g. [α]_{D} = +2.7^{o} (c=1 in Methanol); Schmp.=186^{o}C; DC-Analyse: Rf = 0.43 (Dichlormethan/Aceton 3:1).

### 2-N-(Z)-4-N-(2-acetamido-2-desoxy-3,4,6-tri-O-acetyl-β-D-glucopyranosyl)-L-asparagin tert.-butylester (Verbindung 7)

2-N-(Z)-L-asparaginsäure tert.-butylester (3.1 g), HOBt (1.4 g), und DCCI (2.3 g) wurden in DMF (150 mL) bei 0^{o}C gelöst und dann 1 h gerührt. Nach der Zugabe einer Lösung von Verbindung 3 (3.27 g) in DMF (50 mL) wurde die Reaktionsmischung 40 h bei Raumtemteratur gerührt, dann abfiltriert und eingedampft. Der Rückstand wurde wurde in Dichlormethan gelöst, mit Wasser ausgewaschen und die organische Phase wurde über Natriumsulfat getrocknet und in vacuo eingeengt. Das Rohprodukt wurde säulenchromatographisch über Kieselgel (200 g) mit Dichlormethan und Aceton 4:1 gereinigt. Ausbeute: 3.2 g. DC-Analyse: Rf=0.55 (Dichlormethan/Aceton 2:1).

### 2-N-(Z)-4-N-(2-acetamido-2-desoxy-3,4,6-tri-O-acetyl-β-D-glucopyranosyl)-L-glutamin tert.-butylester (Verbindung 8)

Ausgehend von 2-N-(Z)-L-asparaginsäure tert.-butylester (3.0 g) und Verbindung 2 (3.1 g) wurde die Titelverbindung nach der Vorschrift zur Herstellung der Verbindung 6 hergestellt. Beispiel 3

### Selektive Entblockierung von N-Schutzgruppen der Glycopeptide.

### 4-N-[Methyl-(2,3,4-tri-O-acetyl-β-D-glucopyranosyl)uronat]-L-asparagin-tert.-butyl-ester (Verbindung 9)

Verfahren A: Die N-Fmoc-geschützte Verbindung 4(1.8 g) wurde in DMF (40 mL) gelöst und mit Piperidin (0.4 mL) versetzt. Nach 4 h Rühren wurde die Mischung in vacuo eingedampft, der Rückstand wurde in Ethylacetat und Butanol 5:1 gelöst und mit verdünnter Salzsäure ausgewaschen. Die organische Phase wurde über Natriumsulfat getrocknet und in vacuo eingeengt. Der Rückstand wurde aus Ether und Petrolether kristallisiert. Ausbeute: 0.8 g. Schmp. = 160-163^{o}C
Verfahren B: Die N-Z-geschützte Verbindung 5 (0.9 g) wurde in Ethylacetat und Methanol 1:1(20 mL) gelöst und in der Gegenwart von Pd/C (0.4 g) hydriert. Nach dem Abfiltrieren des Katalysators wurde das Filtrat eingedampft und das erhaltene Produkt wurde ohne weitere Reinigungsschritte in die nächste Reaktion eingesetzt. Ausbeute: 0.75 g.

### 4-N-(2,3,4,6-Tetra-O-acetyl-β-D-glucopyranosyl)-L-asparagin tert.-butylester (Verbindung 10)

Die N-Z-geschützte Verbindung 6(2.2 g) wurde in Ethylacetat/Methanol 1:1(50 mL) gelöst und in der Gegenwart von 10% Pd/C (1.3 g) 4 h hydriert. Die Reaktionsmischung wurde abfiltriert und in vacuo eingedampft. Das erhaltene Produkt wurde ohne weitere Reinigungsschritte für die weitere Umsetzung verwendet. Ausbeute: 1.63 g. DC-Analyse: Rf = 0.45 (Chloroform/Methanol 6:1)

### 4-N-(2-Acetamido-2-desoxy-3,4,6-tri-O-acetyl-β-D-glucopyranosyl)-L-asparagin tert.-butylester. (Verbindung 11)

Die N-Z-geschützte Verbindung 7(2.2 g) wurde in der Gegenwart von Pd/C hydriert und wie oben beschrieben aufgearbeitet. Ausbeute: 1.63 g. DC-Analyse: Rf = 0.47 (Chloroform/Methanol 6:1)

### 4-N-(2-Acetamido-2-desoxy-3,4,6-tri-O-acetyl-β-D-glucopyranosyl)-L-glutamin-tert.-butylester (Verbindung 12)

Die N-Z-geschützte Verbindung 7(2.2 g) wurde in Ethylacetat/Methanol 1:1(50 mL) gelöst und in der Gegenwart von Palladium/Kohle (10%; 1.2 g) 5 h hydriert. Nach Abfiltrieren des Katalysators wurde die Lösung in vacuo eingeengt und der Rückstand wurde zweimal mit Toluol codestilliert. Das erhaltene Produkt (1.32 g) wurde ohne weitere Reinigung für die nachfolgende Umsetzung verwendet.

### Beispiel 4

### Herstellung von aromatischen Sulfonylchloriden

### 2,3,4,6-Tetramethyl-benzolsulfonsäurechlorid (Verbindung 13)

1,2,3,5-Tetramethyl-benzol (5 mL) wurde in Dichlormethan (400 mL) gelöst und auf -10^{o}C abgekühlt. Eine Lösung von Chlorsulfonsäure (6.6 mL) und Dichlormethan (200 mL) wurde zugetropft, die Mischung wurde 3 h gerührt anschließend mit einer 5%-igen Natriumhydrogencarbonat Lösung und mit Wasser ausgewaschen, über Natriumsulfat getrocknet und eingedampft. Das erhaltene Produkt wurde ohne weitere Reinigungsschritte für die nächste Umsetzung eingesetzt. Ausbeute: 7.64 g. DC-Analyse: Rf = 0.71 (Petrolether/Ethylacetat 7:1).

### Beispiel 5

### Herstellung der Sulfonamide

### 2-N-(4-Methoxy-2,3,6-trimethyl-benzolsulfonyl)-4-N-[methyl-(2,3,4-tri-O-acetyl-β-D-glucopyranosyl)uronat]-L-asparagin tert.-butylester (Verbindung 14)

Verbindung 9 (1.73 g), Diisopropylethylamin (1.2 mL) und 4-Methoxy-2,3,6-tri-methyl-benzolsulfonyl chlorid (0.80 g) wurden in DMF (80 mL) gelöst. Die Reaktionsmischung wurde 5 h gerührt und anschließend eingedampft. Der Rückstand wurde in Ethylacetat gelöst und mit Wasser ausgewaschen. Die organische Phase wurde über Natriumsulfat getrocknet und in vacuo eingedampft. Das Rohprodukt wurde sälenchromatographisch über Kieselgel mit Dichlormethan und Aceton 3:1 gereinigt. Ausbeute: 1.57 g. [α]_{D} = +7.0^{o} (c=1 in Methanol); Schmp.=108^{o}C; DC-Analyse: Rf = 0.87 (Dichlormethan/Methanol 10:1).

### 2-N-(4-Methoxy-2,3,6-trimethyl-benzolsulfonyl)-4-N-(2,3,4,6-tetra-O-acetyl-β-D-glucopyranosyl)-L-asparagin tert.-butylester (Verbindung 15)

Verbindung 10 (1.60 g), Diisopropylethylamin (1.0 mL) und 4-Methoxy-2,3,6-tri-methyl-benzolsulfonylchlorid (0.78 g) wurden in DMF (80 mL) gelöst. Die Reaktionsmischung wurde 5 h gerührt und anschließend eingedampft. Der Rückstand wurde in Ethylacetat gelöst und mit Wasser ausgewaschen. Die organische Phase wurde über Natriumsulfat getrocknet und in vacuo eingedampft. Das Rohprodukt wurde sälenchromatographisch über Kieselgel mit Dichiormethan und Aceton 3:1 gereinigt. Ausbeute: 1.57 g. [α]_{D} +7.3^{o} (c 1.0 in Methanol); DC-Analyse: Rf = 0.37 (Dichlormethan und Aceton 3:1).

### 2-N-(4-Methoxy-2,3,6-trimethyl-benzolsulfonyl)-4-N-(2-acetamido-2-desoxy3,4,6-tri-O-acetyl-β-D-glucopyranosyl)-L-asparagin tert.-butylester (Verbindung 16 )

Verbindung 11 (1.6 g) und 4-Methoxy-2,3,6-trimethyl-benzolsulfonsäurechlorid (0.8 g) wurden wie oben beschrieben umgesetzt und aufgearbeitet. Ausbeute: 1.74 g. DC-Analyse: Rf = 0.36 (Dichlormethan/Aceton 3:1)

### 2-N-(4-Methoxy-2,3,6-trimethyl-benzolsulfonyl)-4-N-(2-acetamido-2-desoxy3,4,6-tri-O-acetyl-β-D-glucopyranosyl)-L-glutamin tert.-butylester (Verbindung 17)

Zu einer Lösung der Verbindung 12 (1.6 g) in DMF (80 mL) wurden Diisopropylethylamin (1 mL) und 4-Methoxy-2,3,6-trimethyl-benzolsulfonsäurechlorid (0.76 g) unter pH-Kontrolle (pH> 7.5) zugegeben. Die Mischung wurde 5 h gerührt und in vacuo eingedampft. Der Rückstand wurde in Ethylacetat gelöst und mit Wasser ausgewaschen.Die organische Phase wurde über Natriumsulfat getrocknet und eingeengt. Das erhaltene Produkt wurde über Kieselgel mit Dichlormethan und Aceton 3:1 gereinigt. Ausbeute: 1.60 g. DC-Analyse: Rf = 0.82 (Dichlormethan/Aceton 1:1).

### 2-N-(2,3,4,6-Tetramethyl-benzolsulfonyl)-4-N-(2,3,4,6-tetra-O-acetyl-β-D-glucopyranosyl)-L-asparagin tert.-butylester (Verbindung 18)

Ausgehend von Verbindung 11 und 2,3,4,6-Tetramethylbenzolsulfonsäurechlorid (Verbindung 13) wurde die Titelverbindung nach der Vorschrift zur Herstellung von Verbindung 17 hergestellt.

### Beispiel 6

### Selektive Entblockierung der COO-Schutzgruppen der Glycopeptide.

### 2-N-(4-Methoxy-2,3,6-trimethyl-benzolsulfonyl)-4-N-[methyl-(2,3,4-tri-O-acetyl-b-D-glucopyranosyl)uronat]-L-asparagin (Verbindung 19)

Verbindung 14(2.20 g) wurde in Dichlormethan und Trifluoressigsäure 1:1 (50 mL) gelöst und anschließend 2.5 h bei Raumtemperatur gerührt. Das Reaktionsgemisch wurde eingeengt und der Rückstand wurde mit Toluol codestilliert. Das erhaltene Produkt wurde ohne weitere Reinigungsschritte in die nächste Umsetzung eingesetzt. Ausbeute: 1.4 g. [a]_{D} +38.2^{o} (c 1.0 in Methanol); DC-Analyse: Rf = 0.22 (Dichlormethan/Methanol 10:1)

### 2-N-(4-Methoxy-2,3,6-trimethyl-benzolsulfonyl)-4-N-(2,3,4,6-tetra-O-acetyl-β-D-glucopyranosyl)-L-asparagin (Verbindung 20)

Verbindung 15 (1.5 g) wurde mit Trifluoressigsäure hydrolysiert und wie bei der Verbindung 19 beschrieben aufgearbeitet. Ausbeute: 1.0 g. DC-Analyse: Rf = 0.07 (Chloroform/Methanol/Eisessig 5:1:0.1)

### 2-N-(4-Methoxy-2,3,6-trimethyl-benzolsulfonyl)-4-N-(2-acetamido-2-desoxy3,4,6-tri-O-acetyl-β-D-glucopyranosyl)-L-glutamin (Verbindung 21)

Verbindung 16(1.57 g) wurde mit Trifluoressigsäure hydrolysiert und wie üblich aufgearbeitet. Ausbeute: 1.31 g. DC-Analyse: Rf = 0.3 (Dichlormethan/Aceton 1:1).

### 2-N-(4-Methoxy-2,3,6-trimethyl-benzolsulfonyl)-4-N-(2-acetamido-2-desoxy3,4,6-tri-O-acetyl-β-D-glucopyranosyl)-L-Glutamin Verbindung 22)

Verbindung 17 (1.57 g) wurde in Dichlormethan (60 mL) gelöst und mit Trifluoressigsäure (20 mL) versetzt. Die Mischung wurde 6 h gerührt, in vacuo eingedamft und dreimal mit Toluol codestilliert. Ausbeute: 1.3 g. DC-Analyse: Rf = 0.83 (Dichlormethan/Methanol/Eisessig 5:1:0.5).

### 2-N-(2,3,4,6-Tetramethyl-benzolsulfonyl)-4-N-(2-acetamido-2-desoxy-3,4,6-tri-O-acetyl-β-D-glucopyranosyl)-L-asparagin (Verbindung 23)

Verbindung 18 (0.9 g) wurde wie oben beschrieben mit Trifluoressigsäure hydrolysiert. Ausbeute: 0.6 g. DC-Analyse: Rf = 0.78 (Dichlormethan/Methanol/Eisessig 5:1:0.5).

### Beispiel 7

### Kondensationsschritt

### 2-N-(4-Methoxy-2,3,6-trimethyl-benzolsulfonyl)-4-N-[methyl(-(2,3,4-tri-O-acetyl-β-D-glucopyranosyl)uronat]-L-asparaginyl-4-amidino-D-phenylalanin-piperidid hydrochlorid (Verbindung 24)

Verbindung 19(1.0 g), HOBt (0.23 g) und DCCI (0.37 g) wurden in DMF (50 mL) gelöst und nach 0.5 h Rühren mit 4-Amidino-D-phenylalanin-piperidid (als Salz der Trifluoressigsäure: 0.4 g) versetzt. Die Reaktionsmischung wurde 48 h bei Raumtemperatur gerührt, dann abfiltriert und in vacuo eingedampft. Der Rückstand wurde in Ethylacetat gelöst und mit Wasser ausgewaschen. Das Rohprodukt wurde säulenchromatographisch über Kieselgel mit Chloroform/Methanol 5:1 gereinigt. Ausbeute: 1.2 g. DC-Analyse: Rf = 0.7 (Chloroform/Methanol 3:1).

### 2-N-(4-methoxy-2,3,6-trimethyl-benzolsulfonyl)-4-N-(2,3,4,6-tetra-O-acetyl-β-D-glucopyranosyl)-L-asparaginyl-4-amidino-D-phenylalanin-piperidid hydrochlorid (Verbindung 25)

Verbindung 20 (2.50 g), HOBt (0.65 g) und DCCI (1.15 g) wurden in DMF (100 mL) gelöst und nach 1 h mit 4-Amidino-D-phenylalanin-piperidid hydrochlorid (1:50 g) versetzt. Die Mischung wurde weitere 24 h gerührt und in vacuo eingedampft. Der Rückstand wurde in Chloroform gelöst und mit Wasser ausgewaschen. Die organische Phase wurde in vacuo eingeengt. Das erhaltene Rohprodukt wurde säulenchromatographisch über Kieselgel mit Chloroform und Methanol 3:1 gereinigt.

### 2-N-(4-Methoxy-2,3,6-trimethyl-benzolsulfonyl)-4-N-(2-acetamido-2-desoxy3,4,6-tri-O-acetyl-β-D-glucopyranosyl)-L-asparaginyl-4-amidino-D-phenylalanin-piperidid hydrochlorid (Verbindung 26)

Ausgehend von Verbindung 21 (1.2 g) und 4-Amidino-D-phenylalanin-piperidid hydrochlorid (0.92 g) wurde die Titelvebindung wie oben beschrieben umgesetzt und aufgearbeitet. Ausbeute: 1.32 g DC-Analyse: Rf = 0.47 (Dichlormethan/Methanol/Eisessig 5:1:0.5)

### 2-N-(4-Methoxy-2,3,6-trimethyl-benzolsulfonyl)-4-N-(2-acetamido-2-desoxy 3,4,6-tri-O-acetyl-β-D-glucopyranosyl)-L-glutaminyl-4-amidino-D-phenylalanin-piperidid hydrochlorid (Verbindung 27)

Verbindung 22 und 4-Amidino-D-phenylalanin-piperidid hydrochlorid wurden zu der Titelverbindung wie oben beschrieben umgesetzt.

### 2-N-(2,3,4,6-Tetramethyl-benzolsulfonyl)-4-(2-acetamido-2-desoxy-3,4,6-tri-O-acetyl-β-D-glucopyranosyl)-L-asparaginyl-(4-amidino-D-phenylalanin-piperidid hydrochlorid (Verbindung 28)

Verbindung 20 und 4-Amidino-D-phenylalanin-piperidid hydrochlorid wurden zu der Titelverbindung wie oben beschrieben umgesetzt.

### Beispiel 8

### Entblockierung der O- und COO-Schutzgruppen der Glycopeptid-Derivate:

### 2-N-(4-Methoxy-2,3,6-trimethyl-benzolsulfonyl)-4-N-[methyl-(β-D-glucopyranosyl)uronat]-L-asparaginyl-4-amidino-D-phenylalanin-piperidid hydrochlorid (Verbindung 29)

Verbindung 24(0.5 g) wurde in Methanol (50 mL) gelöst und die Lösung wurde mit 1N NaOH auf pH 9 eingestelt. Nach 2 h Rühren bei pH 8.5-9 wurde die Reaktionsmischung mit methanolischer HCl neutralisiert und in vacuo eingedampft. Der Rückstand wurde säulenchromatographisch (Sephadex LH 20) gereinigt. Ausbeute: 0.44 g. DC-Analyse: 0.34 (Chloroform/Methanol/Wasser).

### 2-N-(4-Methoxy-2,3,6-trimethyl-benzolsulfonyl)-4-N-[(β-D-glucopyranosyl)uronsäure]-L-asparaginyl-4-amidino-D-phenylalanin-piperidid hydrochlorid (Verbindung 30)

Eine Lösung der Verbindung 24 (0.9 g) in Chloroform, Methanol und Wasser 2:1:0.05(100 mL) wurde mit Bariumoxid (0.2 g) versetzt. Die Mischung wurde 3 h gerührt, dann mit methanolischer HCl auf den pH-Wert von 3.5 eingestellt und in vacuo eingedampft. Das erhaltene Produkt wurde säulenchromatographisch (Sephadex LH 20) gereinigt. Ausbeute: 0.72 g. DC-Analyse: Rf = 0.32 (Chloroform/Methanol/Wasser 8:6:1). [α]_{D} = +3.8^{o} (c 0.5 in Methanol)

### 2-N-(4-Methoxy-2,3,6-trimethyl-benzolsulfonyl)-4-N-(β-D-glucopyranosyl)-L-asparaginyl-4-amidino-D-phenylalanin-piperidid hydrochlorid (Verbindung 31)

Eine Lösung der Verbindung 25 (1.2 g) in Chloroform/Methanol 2:1(100 mL) wurde mit Bariumoxid (0.25 g) versetzt und die Mischung wurde 2 h gerührt und dann abfiltriert Das Filtrat wurde mit methanolischer HCl neutralisiert und in vacuo eingedampft. Der Rückstand wurde säulenchromatographisch (Sephadex LH 20) gereingt Ausbeute: 1.0 g. DC-Analyse: Rf = 0.04 (Chloroform/Methanol 3:1)

### 2-N-(4-Methoxy-2,3,6-trimethyl-benzolsulfonyl)-4-N-(2-acetamido-2-desoxy-β-D-glucopyranosyl)-L-asparaginyl-4-amidino-D-phenylalanin-piperidid hydrochlorid (Verbindung 32)

Verbindung 26 (1.0 g) wurde mit Bariumoxid (210 mg) in Chloroform und Methanol (90 mL) wie oben beschrieben deacetyliert. Ausbeute: 0.65g. [a]_{D} = +15.6^{o} (c 1.013 in Methanol)

### 2-N-(4-Methoxy-2,3,6-trimethyl-benzolsulfonyl)-4-N-(2-acetamido-2-desoxy-β-D-glucopyranosyl)-L-glutaminyl-4-amidino-D-phenylalanin-piperidid hydrochlorid (Verbindung 33)

Verbindung 27 wurde mit Bariumoxid wie oben beschrieben deacetyliert.

### 2-N-(2,3,4,6-Tetramethyl-benzolsulfonyl)-4-(2-acetamido-2-desoxy-β-D-glucopyranosyl)-L-asparaginyl-(4-amidino-D-phenylalanin-piperidid hydrochlorid (Verbindung 34)

Verbindung 28 wurde mit Bariumoxid wie oben beschrieben deacetyliert.

### Beispiel 9

### Synthese von p-Amidino-D-phenylalanin-morpholinid

### N-(tert. Butyloxycarbonyl)-D-(p-cyano)phenylalanin-morpholinid (Verbindung 35)

N-(tert. Butyloxycarbonyl)-D-(p-cyano)phenylalanin (5.0 g) wurde in DMF (50 mL) gelöst und mit HOBt (2.6 g) und DCCI (4.2) bei O^{o}C versetzt und 1 h gerührt. Dann wurde die Mischung mit Morpholin (1.5 mL) versetzt, weitere 3 h bei Raumtemperatur gerührt, abfiltriert und in vacuo eingedampft. Der Rückstand wurde in Ethylacetat gelöst und mit einer 5%-igen NaHCO₃-Lösung und einer 5%-igen KHSO₄-Lösung je dreimal ausgewaschen. Die organische Phase wurde noch mit gesättigter Kochsalz-Lösung ausgewaschen, dann über Natriumsulfat getrocknet und in vacuo eingeengt. Ausbeute: 6.5 g. DC-Analyse: Rf=0.83 (Dichlormethan/Methanol/Eisessig 10:1:0.5)

### N-(tert.-Butyloxycarbonyl)-D-(p-thiocarbarmoyl)phenylalanin-morpholinid (Verbindung 36)

In einer Lösung von Verbindung 35(6.5 g) in Pyridin (120 mL) und Triethylamin (3.2 mL) wurde H₂S-Gas 3 h eingeleitet. Die Reaktionsmischung wurde in einem abgeschlossenem Kolben noch 2 Tage gerührt dann mit Toluol versetzt und in vacuo eingedampft. Der Rückstand wurde in Ethylacetat gelöst und mit einer 5%-igen KHSO₄-Lösung ausgewaschen. Die organische Phase wurde über Natriumsulfat getrocknet und in vacuo eingeengt. Das Rohprodukt wurde aus Ethylacetat und Diethylether kristallisiert. Ausbeute: 6.7 g. [a]_{D} = - 11.3^{o}(c 0.743 in Chloroform).

### N-(tert.-Butyloxycarbonyl)-D-(p-methylthioimidino)phenylalanin-morpholinid hydroiodid (Verbindung 37)

Eine Lösung von Verbindung 36 (6.5 g) und Methyliodid (5.2 mL) in Aceton (150 mL) wurde 0.5 h unter Rückfluß gehalten. Der nach dem Abfiltrieren des Reaktionsgemisches erhaltene Niederschlag wurde in Ethylacetat gelöst und mit Eiswasser ausgewaschen. Die organische Phase wurde über Natriumsulfat getrocknet und in vacuo eingedampft. Ausbeute: 7.2 g. [a]_{D}-13.5^{o} (c 1.36 in Dichlormethan); DC-Analyse: Rf=0.53 (Dichlormethan/Methanol/Eisessig 10:1:0.5).

### N(tert.-Butyloxycarbonyl)-D-(p-amidino)phenylalanin-morpholinid hydroiodid (Verbindung 38)

Verbindung 37(5.2 g) wurde in Methanol (80 mL) gelöst und mit Ammoniumacetat (1.0 g) versetzt. Die Mischung wurde bei 60°C 3 h gerührt und eingedampft. Der Rückstand wurde säulenchromatographisch über LH 20 gereinigt. Ausbeute: 4.2 g. [α]_{D} +7.9^{o} (c 1.45 in Methanol)

### N-(tert.-Butyloxycarbonyl)-D-(p-amidino)phenylalanin-morpholinid Trifluoressigsäure Salz (Verbindung 39)

Verbindung 38 (4.0 g) wurde in Methanol und Wasser 10:1 gelöst und Natrium trifluoracetat (5.0 g) versetzt. Die Mischung wurde 24 h gerührt, abfiltriert und in vacuo eingedampft. Der Rückstand wurde über Sephadex LH 20 Träger filtriert. Ausbeute: 3.2 g.

### D-(p-Amidino)phenylalanin-morpholinid Trifluoressigsäure Salz (Verbindung 40)

Verbindung 39 (3.2 g) wurde mit Trifluoressigsäure nach üblichen Verfahren hydrolysiert und aufgearbeitet. Ausbeute: 2.2 g.

### Beispiel 10

### 2-N-(4-Methoxy-2,3,6-trimethyl-benzolsulfonyl)-4-N-(β-D-galactopyranosyl)-L-asparaginyl-4-amidino-D-phenylalanine-piperidid hydrochlorid (Verbindung 41)

2-N-(4-Methoxy-2,3,6-trimethyl-benzolsulfonyl)-L-asparaginyl-4-amidino-D-phenylalanine-piperidid hydrochlorid (1.0 g), HOBt (0.23 g) und DCCI (0.37 g) wurden in DMF und Dichlormethan (1:1(50 mL) gelöst und nach 0.5 h Rühren mit b-D-galactopyranosylamin (1.0 g) versetzt. Die Reaktionsmischung wurde 48 h bei Raumteperatur gerührt, abfiltriert und in vacuo eingedampft. Der Rückstand wurde in Ethylacetat gelöst und mit Wasser gewaschen. Das Rohprodukt wurde säulenchromatographisch über Kieselgel mit Chloroform/Methanol 5:1 gereinigt. Ausbeute: 1.2 g. DC-Analyse: Rf=0.73 (Chloroform/Methanol 3:1).Das Zwischenprodukt (1.2 g) wurde in Chloroform/Methanol 2:1 (100 mL) gelöst und mit Bariumoxid (0.25 g) versetzt. Die Mischung wurde 2 h gerührt und abfiltriert. Das Filtrat wurde mit methanolischer HCl neutralisiert und in vacuo eingedampft. Der Rückstand wurde säulenchromatographisch über Sephadex LH 20 gereinigt. Ausbeute: 1.0 g. DC-Analyse: Rf=0.042 (Chloroform/Methanol 3:1). [α]_{D} + 19.3^{o} (c=1 in Methanol). Schmp. = 186-190^{o}C.

### Beispiel 11

### Bestimmung der Inhibitionskonstanten für Thrombin

Die Inhibitionskonstanten (Ki) der Verbindungen der Formel I wurden in etablierten enzymkinetischen Verfahren ermittelt. Das eingesetzte humane Thrombin wurde als 87% rein mit Hilfe der "active site titration" bestimmt Die Testlösung für die Ki-Bestimmung bestand aus Puffer (50 mM Tris-HCl, 75 mM NaCl, pH 7,8, 37^{o}C), 100 pM Thrombin, 0,1 nM H-D-Phenyalany-L-pipecolyl-L-arginin-p-nitroanilid dihydrochlorid (Substrat S-2238; Fa. Kabi) und Inhibitor, der einen Bereich von 0 bis 400 nM umspannte. Inhibitor und Enzym wurden 10 Minuten vorinkubiert, die Reaktion wurde gestartet durch Zugabe des chromogenen Substrats S2238. Für die Auswertung der Kinetiken wurde der mathematische Algorithmus für "tight-binding" verwendet, der mit Hilfe der nicht-linearen Regression Ki-Werte und den Hemmtyp ergab. Der Hemmtyp für alle Inhibitoren wurde als kompetitiv ermittelt. Die Ergebnisse sind in der Tabelle I zusammengefaßt.

### Beispiel 12

### Bestimmung der Spezifität der Inhibitoren

Die Spezifität der Inhibitoren wurde gegenüber Thrombin und Trypsin bestimmt. Analog der Ki-Bestimmung von Thrombin wurde der Ki-Wert von Trypsin bestimmt.

Die Reaktionsmischung setzte sich wie folgt zusammen - Puffer: 200 mM Triethanolamin, 20 mM CaCl₂, pH 7.8; 37^{o}C; Enzym: 0.5 nM Trypsin aus Rinderpankreas; Substrat: 500 mM Carbobenzoxy-L-valyl-glycyl-L-arginin-4-nitranilid (Chromozym TRY, Fa. Boeringer Mannheim). Die enzymatische Reaktion wurde gestartet nach 10-minutigen Vorinkubation durch Zugabe des Substrats. Das freigesetzte p-Nitroanilin wurde bei 405 nm gemessen. Die Ergebnisse sind in der Tabelle I zusammengefaßt.

### Beispiel 13

### Ermittlung der akuten Toxizität

Zur Ermittlung der akuten Toxizität wurden CD-Ratten am Tag 0 unterschiedliche Dosen der Testsubstanz gelöst in 0.5 mL physiol. NaCl-Lösung i.v. injiziert. Kontrollgruppen erhielten lediglich 0.5 mL physiol. NaCl Lösung. Pro Konzentration der Testsubstanz wurden 2 Ratten verwendet. Am Tag 1 wurde die Zahl der überlebten Ratten ermittelt und nach der Lichtfield Wilcoxon Methode die LD5, LD50 und LD95 ermittelt. Die Toxizität LD50 (mg/kg) der hier beschriebenen Verbindungen im Vergleich zu NAPAP wurde ermittelt. Die Ergebnisse sind in der Tabelle I zusammengefaßt.

### Pharmakologischen Daten und Wirksamkeiten einiger ausgewählter Verbindungen:

**Tabelle I**

| Verbindung | Ki-Thrombin (nMol/L) | Ki-Trypsin (nMol/L) | Spezifität LD₅₀ Try/Thr | (mg/kg) (Ratte) |
|---|---|---|---|---|
| NAPAP | 11.0 | 499 | 46 | |
| Verbindung 24 | 0.8 | 231 | 289 | 46 |
| Verbindung 25 | 2.4 | 283 | 118 | 55 |
| Verbindung 30 | 0.085 | 349 | 4106 | 51 |
| Verbindung 31 | 0.075 | 501 | 6680 | 45 |
| Verbindung 32 | 1.1 | 528 | 480 | 53 |
| Verbindung 41 | 0.04 | 375 | 9375 | 50 |
| Verbindung 42 | 1.3 | 650 | 500 | 60 |
| Verbindung 43 | 1.5 | 187 | 125 | 62 |
| Verbindung 49 | 0.1 | 531 | 5310 | 52 |
| Verbindung 50 | 2.1 | 608 | 289 | 64 |

### Beispiel 14

### Partial tromboplastin time (PTT) - Test für Defekte des intrinsichen und extrinsichen Coagulatiossystems

Die getesteten Verbindungen wurden i.v. verabreicht als Bolus in die Schwanz wehne der Ratte. Nach 5 min wurde Blut aus der Plexusvene entnommen mit 1/5 Vol. Citrat-Puffer vermischt. In einem Koagulometer (Schnittger & Gross) wurde mit Neothrombin der Wert von PTT beschtimmt. Der normale PTT-Wert tiegt bei der Ratte im Bereich von 20 sec. Für die Bestimmung von Trombin time (TT) wurde 100 mL von Blut, Citrat-Puffer und Diethylbarbiturat/Acetat-Puffer vermischt und 1 min bei 37^{o}C inkubiert. Nach der Zugabe von 100 mL Test-Thrombin wurde der TT-Wert in einem Coagulometer bestimmt.

### Beispiel 15

### Synthese von 2-N-(4-Methoxy-2,3,6-trimethylbenzolsulfonyl)-3-O-(β-D-ribopyranosyl)-L-seryl-4-amidino-D-phenylalanin-piperidid hydroiodid (Verbindung 42)

Stufe 1: Synthese von 2-N-(4-Methoxy-2,3,6-trimethylbenzolsuifonyl)-3-O-(β-D-ribopyranosyl)-L-seryl-4-cyano-D-phenylalanin-piperidid hydroiodid: 3-O-(β-D-ribopyranosyl)-L-serin-tert. butyl ester (17.3 g), triethylamin (12 mL) und Mtr-chlorid wurden in DMF (800 mL) gelöst. Die Lösung wurde 16 h gerührt und dann in vacuo eingedampft. Der Rückstand wurde in Ethylacetat gelöst, die Lösung wurde dreimal mit Wasser ausgewaschen, dann über Natrium sulfat getrocknet und in vacuo eingedampft. Der Rückstand wurde chromatographisch über Kieselgel mit dichlormethan/acetone(3:1/V:V). Ausbeute: 15.7 g. DC-Analyse: Rf = 0.87 (dichlormethan/methanol 10:1). Das intermediate wurde in 300 mL trifluoressigsäure/dichlormethan (1:1) gelöst und die Lösung wurde 1 h bei Raumtemperatur gerührt. Die sauere lösung wurde in vacuo eingedampft und der Rückstand wurde mit Toluol codeslliert. Das resultierende Produkt wurde ohne weitere Reinigung in die nächste Stufe eingesetzt. Das Rohprodukt (10 g), HOBt (2.3 g) und DCCI (3.7 g ) wurden in 500 mL DMF gelöst und die Mischung wurde bei 4^{o}C für 30 min gerührt. Anschließend wurden 4-cyano-D-phenylalanyl-piperidin (9.0 g) und N-methylmorpholin (5.0 mL) zugegeben. Die Mischung wurde 18 h bei Rautemperatur gerührt, abfiltriert und in vacuo eingedampft. Das Rohprodukt wurde säulenchromatographisch über Kieselgel mit chloroform/methanol 5:1 und ethyl acetat gereinigt. Ausbeute: 14.3 g. DC-Analyse Rf=0.86 (chloroform/methanol 10:1).
Stufe 2: Synthese von 2-N-(4-Methoxy-2,3,6-trimethyl-benzolsulfonyl)-3-O-(2,3,4-tri-O-acetyl-β-D-ribopyranosyl)-L-seryl-4-amidino-D-phenylalanyl-piperidin x HI: Cyanophenylalanin glycopeptid (10.0 g) wurde in trockenem Pyridine (100 mL) gelöst und nach der Zugabe von Triethylamin (2.5 mL), H₂S-Gas wurde für 3 h eingeleitet. Die Mischung wurde 3 d bei Raumtemperatur stehengelassen und dann in eine HCl-Eislösung (300 g) gegossen. Das Präzipitat wurde abfiltriert und mit Wasser nachgewaschen. Das Thioamide wurde getrocknet, dann in Aceton (200 mL) gelöst. Nach der Zugabe von Methyl iodide (5.0 mL) wurde die MIschung 30 min unter Rückfluß gehalten. Die abgekühle Mischung wurde anschließend mit Ether präzipitiert. Das in Dichlormethangelöste Präzipitat wurde zweimal mit Wasser gewaschen. Die organische Phase wurde über Natriumsulfat getrocnet und in vacuo eingedampft. Der Rückstand (8.2 g) wurde in Methanol (200 mL) gelöst und mit Ammonium acetat (2.1 g) versetzt. Die Mischung wurde bei 60^{o}C 3 h gerührt und dann in vacuo eingedampft. Das Rohprodukt wurde säulenchromatographisch über Sephadex LH-20 mit Methanol und anschließend über Kieselgel mit Chloroform/Methanol/Wasser 10:1:0.2 gereinigt. Ausbeute: 6.2 g. DC-Analyse: Rf = 0.3 (Chloroform/Methanol/Wasser 10:1:0.2).
Stufe 3: Deacylierung von 2-N-(4-Methoxy-2,3,6-trimethyl-benzolsulfonyl)-3-O-(2,3,4-tri-O-acetyl-β-D-ribopyranosyl)-L-seryl-4-amidino-D-phenylalanyl-piperidin x HI: Die acylierte Verbindung (1.8 g) wurde in chloroform, methanol and water 2:1:0.05(200 mL) gelöst und mit Barium oxid (0.4 g) versetzt. Die Mischung wurde 3 h gerührt. Nach der Einstellung des pH-Wertes auf pH3.5 mitmethanolischerHCl wurde die Lösung in vacuo eingedampft. Das erhaltene Produkt wurde säulenchromatographisch über Sephadex LH 20 gereinigt. Ausbeute: 1.42 g (Verbindung 42). DC-Analyse: Rf = 0.22 (Chloroform/Methanol/Wasser 8:1:0.2).

### Beispiel 16

### Synthese von 2-N-(4-Methoxy-2,3,6-trimethyl-benzolsulfonyl)-L-Asp-4-amidino-D-phenylalanine-piperidid x HCl (Verbindung 43)

Verbindung 43 wurde, wie in der deutschen Offenlegungsschrifft DE 4115468 A1 beschrieben hergestellt.

### Beispiel 17

### Herstellung von 2-N-(Urethan-Schutzgruppe)-Asp-α-estern ausgehend von 2-N-(Urethan-Schutzgruppe)-Asp-β-methyl estern 2-N-BOC-L-asparagin-α-benzyl ester (Verbindung 44)

N-BOC-L-Asp-β-O-methyl ester (5.0 g) wurde in Benzylalkohol (40 mL) gelöst und mit Kalium O-tert.-butylate versetzt. Die Lösung wurde 8 h bei 60^{o}C gerührt. Die abgekühlte Lösung wurde zweimal mit Wasser gewaschen, mit Natriumsulfat getrocknet und eingedampft. Der Rückstand wurde mit Diethylether/Petrolether kristallisiert. Ausbeute: 4.5 g.

### 2-N-Benzyloxycarbonyl-L-asparagin-α-benzyl ester (Verbindung 45)

Ausgehend von N-Z-L-Asp-β-O-methyl ester (5.0 g) wurde die die Titelverbindung wie oben beschrieben hergestellt.

### 2-N-Benzyloxycarbonyl-L-asparagin-α-n-butyl ester (Verbindung 46)

N-Z-L-Asp-β-O-methyl ester (5.0 g) wurde in n-Butylalkohol (40 mL) gelöst und mit Kalium O-tert.-butylate versetzt. Die Mischung wurde 8 h bei 60^{o}C gerührt. Die abgekühle Mischung wurde zweimal mit Wasser gewaschen, über Natriumsulfat getrocknet und eingedampft. Der Rückstand wurde mit Diethylether/Petrolether kristallisiert. Ausbeute: 4.5 g.

### Beispiel 18

### Herstellung von 2-N-(Urethan-Schutzgruppe)-4-N-glycosyl-asparagin-α-estern, 2-N-(Urethan-Schutzgruppe)-5-N-glycosyl-glutamin-α-estern und 2-N-(Urethan-Schutzgruppe)-4-N-glycosyl-4-amido-phenylalanine estern

### 2-N-BOC-4-N-β-L-Fucopyranosyl-L-asparagin-α-benzyl ester (Verbindung 47)

Ausgehend von Verbindung 44 und β-L-Fucopyranosyl-amin wurde die Titelverbindung wie im Beispiel 2 beschrieben hergestellt.

### 2-N-Fmoc-4-N-(2-Acetamido-2-desoxy-β-D-glucopyranosyl)-4-acetamido-L-phenylalanin-α-benzylester (Verbindung 48)

Ausgehend von 2-N-Fmoc-4-Carboxy-L-phenylalanin-α-benzylester und 2-Acetamido-2-desoxy-β-D-glucopyranosyl-amin wurde die Titelverbindung wie im Beispiel 2 beschrieben hergestellt.

### Beispiel 19

### 2-N-(4-Methoxy-2,3,6-trimethyl-benzolsulfonyl)-4-N-(β-L-fucopyranosyl)-L-aspaginyl-4-amidino-D-phenylalanine-piperidide hydrochloride (Verbindung 49)

Verbindung 47 wurde mit Trifluoressigsäure/Dichloromethan wie im Beispiel 6 für die Verbindun 19 beschrieben entblockiert. Das Zwischenprodukt wurde mit Mtr-chlorid wie im Beispiel 5 beschrieben umgesetzt. Die erhaltene Verbindung (Mtr-4-N-glycosyl-Asn-OH) wurde mit 4-Amidin-D-phenylalanine-piperidid hydrochloride zu der Titelverbindung 49 wie oben beschrieben (Beispiel 7) umgesetzt und aufgearbeitet.

### Beispiel 20

### 2-N-(4-Methoxy-2,3,6-trimethyl-benzolsulfonyl)-4-N-(2-acetamido-2-desoxy-β-D-galactopyranosyl)-L-aspaginyl-4-amidino-D-phenylalanin-piperidid hydrochlorid (Verbindung 50)

Stufe 1: Synthese von 2-N-(4-Methoxy-2,3,6-trimethyl-benzolsulfonyl)-L-Asp-α-O-benzylester
   2-N-(4-Methoxy-2,3,6-trimethyl-benzolsulfonyl)-L-Asp-β-O-methylester (5.0 g) wurde in benzyl alkohole (40 mL) gelöst und mit Kalium O-tert.-butylat versetzt. Die Lösung wurde 8 h bei 60^{o}C gerührt und nach dem Abkühlen zweimal mit Wasser gewaschen. Die organische Phase wurde über Natriumsalfat getrocknet und in vacuo eingedampft. Das Rohprodukt wurde in Diethylether/Petrolether kristallisiert. Ausbeute: 4.5 g. [α]_{D} = + 43.3^{o} (c=1 in MeOH).
Stufe 2: N-Glycosylierung:
   Das Vorprodukt (4.5 g) und 2-Acetamido-2-desoxy-β-D-galactopyranosyl-amin wurden zu 2-N-(4-Methoxy-2,3,6-trimethyl-benzolsulfonyl)-4-N-(2-acetamido-2-de soxy-β-D-galactopyranosyl)-L-aspagin-α-O-benzylester wie im Beispiel 7 beschrieben umgesetzt. Ausbeute: 4.6 g.
Stufe 3: Hydrierung
   Das Vorprodukt (4.6 g) wurde in der Gegenwart von Pd/C (2 g) in Ethylacetate/Methanol 1:1 hydriet und wie üblich aufgearbeitet. Die erhaltene Verbindung Mtr-(2-acetamido-2-desoxy-β-D-galactopyranosyl)-L-aspagin (3.55 g) wurde ohne weitere Reinigungsschritte in die nächste Stufe eingesetzt.
Stufe 4: Kondensationsstufe:
   Ausgehend von Mtr-(2-acetamido-2-desoxy-β-D-galactopyranosyl)-L-aspagin ( 3.55 g) und 4-Amidino-D-phenylalanine-piperidid hydrochlorid (3.3 g) wurde die Titelverbindung 44 wie im Beispiel 7 beschrieben hergestellt und aufgearbeitet. Ausbeute der Verbindung 50:4.4 g. DC-Analyse: Rf = 0.47 (Dichlormethan/Methanol/Eisessig 5:1:0.5), [α]_{D} = + 21.2^{o} (c=1 in MeOH).

### Beispiel 21

### Herstellung von 2-N-(Urethan-Schutztgruppe)-4-carboxy-phenylalanin und deren Estern

### N-Fmoc-4-tert.-butyloxycarbonyl-D and L-phenylalanin-α-benzylester (Verbindungen 51 und 52)

N-Benzyloxycarbonyl-4-cyano-D und L-phenylalanin-a-benzylester (5.0 g) wurde mit H₂S, dann mit Methyl iodide, wie im Beispiel 15, Stufe 2 beschrieben umgesetzt, wobei die S-Methyl-thioimide-Verbindung (4.2 g) entstanden ist. Das Zwischenprodukt wurde mit wäßriger 5%-iger KHSO₄/Acetone-Lösung wie üblich umgesetzt und aufgearbeitet. Die erhaltene S-Methyl-thioester-Verbindung wurde nach dem Zemplen-Verfahren zu N-Benzyloxycarbonyl-4-carboxy-phenylalanin-α-benzyl ester umgesetzt. Nach dem Schutzt der Carboxygruppe mit tert.-Butyl-Schutzgruppe und folgender Hydrogenolyse wurde die Zwischenverbindung mit Fmoc-Chloride umgesetzt, wobei die Titelverbindungen 51 oder 52 entstanden sind.

## Patentansprüche

1. O-und N-Glycopeptid-Derivate der Formel I 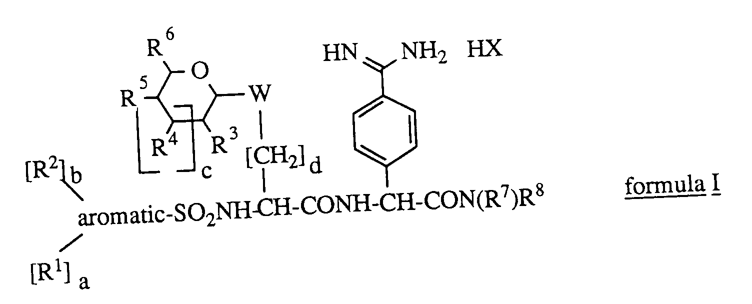 worin
Aromat Benzol-, Naphthalin-, Chroman-, Chromen- oder Cumaron-Rest,
a 1 bis 5, b 0 bis 4, c 0 oder 1, d 1 oder 2 und die Reste
R¹ H oder (C₁-C₃)-Alkyl,
R² H, (C₁-C₃)-Alkyl oder (C₁-C₃)-Alkyloxy,
R³ H, OH, (C₁-C₃)-Alkyloxy, NH₂, NH-(C₁-C₆)-Alkanoyl, NH-Benzoyl, NH-SO₃H oder NH-Acylrest einer natürlichen N-acetylierten Aminosäure,
R⁴ H, OH, oder (C₁-C₃)-Alkyloxy,
R⁵ H, OH, (C₁-C₃)-Alkyloxy, Fluor, Chlor oder Brom,
R⁶ H, CH₃, CH₂OH, CH₂O-(C₁-C₆)-Alkanoyl, CH₂NHCOCH₃ oder CH₂NH-SO₃H
R⁵ und R⁶ zusammen O-CH₂-O-CH₂, O-CH(CH₃)-O-CH₂ oder O-C(CH₃)₂-O-CH₂,
R⁷ Hydroxy-(C₂-C₄)-alkyl oder (C₂-C₄)-Alkyloxy-(C₂-C₄)-alkyl,
R⁸ H oder (C₁-C₆)-Alkyl oder
R⁷-N-R⁸ zusammen einen Pyrrolidin-, Piperidin- oder Morpholinring bilden, der mit den Substituenten HO, HOCH₂, CH₃ oder COOH werden kann,
W -O-, -CONH- oder -C₆H₄CONH- und
H-X HCl, (C₁-C₇)-Alkansäure oder eine physiologisch verträgliche anorganische oder organische Säure,
sind.

2. O- und N-Glycopeptid-Verbindungen nach Anspruch 1,
worin
Aromat Benzol-, Naphthalin- oder Chroman-Rest,
a 1 bis 4, b 0 bis 2, c 0 oder 1, d 1 oder 2 und die Reste
R¹ H oder CH₃,
R² H, CH₃ oder CH₃O,
R³ H, OH, (C₁-C₃)-Alkyloxy, NH₂, NH-Acetyl, NH-Benzoyl, NH-SO₃H oder NH-Acylrest von N-Acetyl-glycin oder N-Acetyl-alanin,
R⁴ H oder OH,
R⁵ H oder OH,
R⁶ H, CH₃, CH₂OH, CH₂O-(C₁-C₆)-Alkanoyl, CH₂NHCOCH₃, CH₂NH-SO₃H, oder CONH(C₁-C₄)-Alkyl,
R⁵ und R⁶ zusammen O-CH₂-O-CH₂ oder O-CH(CH₃)-O-CH₂,
R⁷ Hydroxypropyl oder Ethoxypropyl,
R⁸ H oder (C₁-C₆)Alkyl oder
R⁷-N-R⁸ zusammen einen Pyrrolidin-, Piperidin- oder Morpholinring bilden,
W -O-, -CONH- oder -C₆H₄CONH- und
H-X HCI, (C₁-C₆)-Alkansäure oder eine pharmakologisch vertretbare anorganische oder organische Säure, sind.

3. O- und N-Glycopeptid-Verbindungen nach Anspruch 1,
worin
Aromat Benzol-Rest,
a 1 bis 4, b 0 bis 2, c 0 oder 1, d 1 oder 2 und die Reste
R¹ H oder CH₃,
R² H, CH₃ oder CH₃O,
R³ H, OH, (C₁-C₃)-Alkyloxy, NH₂, NH-Acetyl, NH-Benzoyl, NH-SO₃H oder NH-Acylrest von N-Acetyl-glycin oder N-Acetyl-alanin,
R⁴ H oder OH,
R⁵ H oder OH,
R⁶ H, CH₃, CH₂OH, CH₂O-(C₁-C₆)-Alkanoyl, CH₂NHCOCH₃, CH₂NH-SO₃H, oder CONH(C₁-C₄)-Alkyl,
R⁵ und R⁶ zusammen O-CH₂-O-CH₂ oder O-CH(CH₃)-O-CH₂,
R⁷ Hydroxypropyl oder Ethoxypropyl,
R⁸ H oder (C₁-C₆)-Alkyl oder
R⁷-N-R⁸ zusammen einen Pyrrolidin-, Piperidin- oder Morpholinring bilden,
W -O-, -CONH- oder -C₆H₄CONH- und
H-X HCl, (C₁-C₆)-Alkansäure oder eine pharmakologisch vertretbare anorganische oder organische Säure,
sind

4. O- und N-Glycopeptid-Verbindungen nach Anspruch 1,
worin
Aromat Naphthalin-Rest,
a 1 bis 4, b 0 bis 2, c 0 oder 1, d 1 oder 2 und die Reste
R¹ H oder CH₃,
R² H, CH₃ oder CH₃O,
R³ H, OH, (C₁-C₃)-Alkyloxy, NH₂, NH-Acetyl, NH-Benzoyl, NH-SO₃H oder NH-Acylrest von N-Acetyl-glycin oder N-Acetyl-alanin,
R⁴ H oder OH,
R⁵ H oder OH,
R⁶ H, CH₃, CH₂OH, CH₂O-(C₁-C₆)-Alkanoyl, CH₂NHCOCH₃, CH₂NH-SO₃H, oder CONH(C₁-C₄)-Alkyl,
R⁵ und R⁶ zusammen O-CH₂-O-CH₂ oder O-CH(CH₃)-O-CH₂,
R⁷ Hydroxypropyl oder Ethoxypropyl,
R⁸ H oder (C₁-C₆)-Alkyl oder
R⁷-N-R⁸ zusammen einen Pyrrolidin-, Piperidin- oder Morpholinring bilden,
W -O-, -CONH- oder -C₆H₄CONH- und
H-X HCl, (C₁-C₆)-Alkansäure oder eine pharmakologisch vertretbare anorganische oder organische Säure, sind.

5. O- und N-Glycopeptid-Verbindungen nach Anspruch 1,
worin
Aromat Chroman-Rest,
a 1 bis 4, b 0 bis 2, c 0 oder 1, d 1 oder 2 und die Reste
R¹ H oder CH₃,
R² H,CH₃ oder CH₃O,
R³ H, OH, (C₁-C₃)-Alkyloxy, NH₂, NH-Acetyl, NH-Benzoyl, NH-SO₃H oder NH-Acylrest von N-Acetyl-glycin oder N-Acetyl-alanin,
R⁴ H oder OH,
R⁵ H oder OH,
R⁶ H, CH₃, CH₂OH, CH₂O-(C₁-C₆)-Alkanoyl, CH₂NHCOCH₃` CH₂NH-SO₃H, oder CONH(C₁-C₄)-Alkyl,
R⁵ und R⁶ zusammen O-CH₂-O-CH₂ oder O-CH(CH₃)-O-CH₂,
R⁷ Hydroxypropyl oder Ethoxypropyl,
R⁸ H oder (C₁-C₆)-Alkyl oder
R⁷-N-R⁸ zusammen einen Pyrrolidin-, Piperidin- oder Morpholinring bilden,
W -O-, -CONH- oder -C₆H₄CONH- und
H-X HCl, (C₁-C₆)-Alkansäure oder eine pharmakologisch vertretbare anorganische oder organische Säure,
sind

6. Verfahren zur Herstellung einer Verbindung nach Anspruch 1,
**dadurch gekennzeichnet,**
daß man eine Verbindung der Formel II, 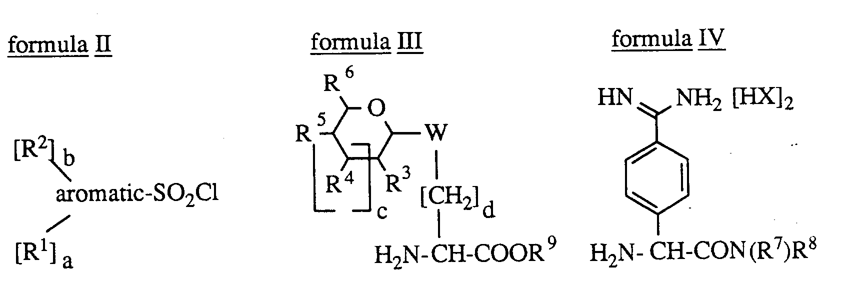 worin
Aromat Benzol-, Naphthalin-, Chroman-, Chromen- oder Cumaron-Rest,
a 1 bis 5, b 0 bis 4, und die Reste
R¹ H oder (C₁-C₃)Alkyl,
R² H, (C₁-C₃)-Alkyl oder (C₁-C₃)-Alkyloxy bedeuten,
mit einem N-Glycosid der Formel III,
worin
c 0 oder 1, d 1 oder 2
R³ H, O-Schutzgruppe, NH-Schutzgruppe, NH-SO₃H, (C₁-C₃)-Alkyloxy, NH-(C₁-C₆)-Alkanoyl, NH-Benzoyl, NH-SO₃H oder NH-Acylrest einer natürlichen N-acetylierten Arminosäure,
R⁴ H, O-Schutzgruppe oder (C₁-C₃)-Alkyloxy,
R⁵ H, O-Schutzgruppe, (C₁-C₃)-Alkyloxy, Fluor, Chlor oder Brom,
R⁶ H, CH₃, CH₂-O-Schutzgruppe, CH₂O-(C₁-C₆)-Alkanoyl, CH₂NHCOCH₃ oder CH₂NH-SO₃H,
R⁵ und R⁶ zusammen O-CH₂-O-CH₂, O-CH(CH₃)-O-CH₂ oder O-C(CH₃)₂-O-CH₂,
R⁹ Methyl-, tert.-Butyl-, Allyl- oder Benzyl-Schutzgruppe und
W -O-, -CONH- oder -C₆H₄CONH-
in Gegenwart einer organischen Base und eines organischen Lösungsmittels zu einer Sulfonamid-Verbindung der Formel V, 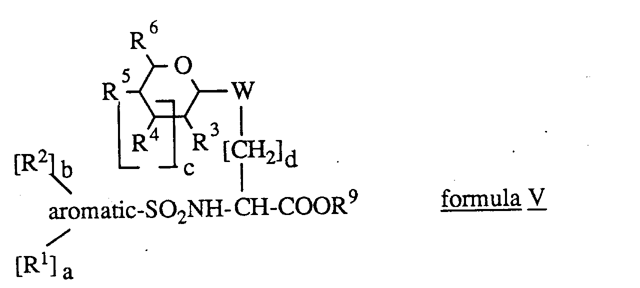 wobei
a, b, c und d und die Reste ihre vorher genannte Bedeutung beibehalten, umsetzt, in dem Produkt selektiv den Rest R⁹ hydrolytisch mit HCl/Essigsäure oder Trifluoressigsäure/Wasser oder hydrogenolytisch in Gegenwart von Palladium/Kohle und (C₁-C₄)-Alkohols, Essigsäure oder Ethylacetat als Lösungsmittels abspaltet, und das entstandene Produkt, worin R⁹ ein Wasserstoffatom ist, mit einem Phenylalaninderivat der Formel IV,
worin
R⁷ Hydroxy-(C₂-C₄)-alkyl oder (C₂-C₄)-Alkyloxy-(C₂-C₄)-alkyl,
R⁸ H oder (C₁-C₆)-Alkyl oder
R⁷-N-R⁸ zusammen einen Pyrrolidin-, Piperidin- oder Morpholinring bilden und
H-X HCl, HI, CF₃COOH sind,
nach einem in der Peptidchemie üblichen Kondensationsverfahren zu einer Verbindung der Formel I,
wobei
a, b, c und d und die Reste ihre vorher genannte Bedeutung beibehalten, umsetzt und in dem Produkt nach in der Kohlenhydrat- oder Peptidchemie üblichen Verfahren die Schutzgruppen entfernt, wobei ein weiteres Produkt der Formel I,
worin
Aromat Benzol-, Naphthalin-, Chroman-, Chromen- oder Cumaron-Rest,
a 1 bis 5, b 0 bis 4, c 0 oder 1, d 1 oder 2 und die Reste
R¹ H oder (C₁-C₃)-Alkyl,
R² H, (C₁-C₃)-Alkyl oder (C₁-C₃)-Alkyloxy,
R³ H, OH, (C₁-C₃)-Alkyloxy, NH₂, NH-(C₁-C₆)-Alkanoyl, NH-Benzoyl, NH-SO₃H oder NH-Acylrest einer natürlichen N-acetylierten Aminosäure,
R⁴ H, OH, oder (C₁-C₃)Alkyloxy,
R⁵ H, OH, (C₁-C₃)-Alkyloxy, Fluor, Chlor oder Brom,
R⁶ H, CH₃, CH₂OH, CH₂O-(C₁-C₆)-Alkanoyl, CH₂NHCOCH₃ oder CH₂NH-SO₃H,
R⁵ und R⁶ zusammen O-CH₂-O-CH₂, O-CH(CH₃)-O-CH₂ oder O-C(CH₃)₂-O-CH₂,
R⁷ Hydroxy-(C₂-C₄)-alkyl oder (C₂-C₄)-Alkyloxy-(C₂-C₄)-alkyl,
R⁸ H oder (C₁-C₆)-Alkyl oder
R⁷-N-R⁸ zusammen einen Pyrrolidin-, Piperidin- oder Morpholinring bilden,
W -O-, -CONH- oder -C₆H₄CONH- und
H-X HCl, (C₁-C₆)-Alkansäure oder eine pharmakologisch vertretbare anorganische oder organische Säure, sind,
entsteht, und das so erhaltene Glycopeptid-Derivat gegebenfalls in sein physiologisch verträgliches Salz überführt.

7. Verbindungen nach Anspruch 1 als Arzneimittel.
